# EUROPEAN PATENT SPECIFICATION

(11) **EP 3 229 665 B1**
(45) Date of publication and mention of the grant of the patent: **20.05.2020**
(21) Application number: 15866619.8
(22) Date of filing: 11.11.2015
(51) Int. Cl.: A61B 5/0408, A61B 5/04, H04B 13/00, A61B 5/0476

(54) **TECHNOLOGIES FOR BIOFEEDBACK ELECTRODE CONTACT MONITORING**
TECHNOLOGIEN ZUR ÜBERWACHUNG EINES BIOFEEDBACK-ELEKTRODENKONTAKTES
TECHNOLOGIES DE SURVEILLANCE DE CONTACT D'ÉLECTRODE PAR RÉTROACTION BIOLOGIQUE

(30) Priority: 11.12.2014 US 201414567136
(43) Date of publication of application: 18.10.2017
(73) Proprietor: Intel Corporation, Santa Clara, CA 95054 (US)
(72) Inventor: FALCONER, Maynard C., Portland, Oregon 97229 (US)
(74) Representative: Rummler, Felix
(86) International application number: PCT/US2015/060067
(87) International publication number: WO 2016/094014

(56) References cited:
- WO-A1-2009/147615
- WO-A1-2014/027298
- JP-A- 2006 141 493
- JP-A- 2012 205 632
- US-A1- 2010 315 206
- US-A1- 2011 237 904
- US-A1- 2011 306 469
- US-A1- 2012 136 346
- US-A1- 2014 296 682
- SEULKI LEE ET AL: "Low Power and Self-Reconfigurable WBAN Controller for Continuous Bio-Signal Monitoring System", IEEE TRANSACTIONS ON BIOMEDICAL CIRCUITS AND SYSTEMS, IEEE, US, vol. 7, no. 2, 1 April 2013 (2013-04-01), pages 178-185, XP011507015, ISSN: 1932-4545, DOI: 10.1109/TBCAS.2013.2254116

## Description

### CROSS-REFERENCE TO RELATED U.S. PATENT APPLICATION

The present application claims priority to U.S. Utility Patent Application Serial No. 14/567,136, entitled "TECHNOLOGIES FOR BIOFEEDBACK ELECTRODE CONTACT MONITORING," which was filed on December 11, 2014.

### BACKGROUND

Typical biofeedback monitoring devices for measuring biofeedback signals, also commonly referred to as biosignals, of a user include sensors and electrodes in contact with the user to capture and measure the biofeedback signals. Examples of biofeedback monitoring devices include electroencephalogram (EEG) devices, electrocardiogram (ECG) devices, and electromyogram (EMG) devices. The electrodes of the biofeedback monitoring devices may be used to sense electrical activity of organs, such as the brain via an EEG device, the heart via an ECG device, or skeletal muscle using an EMG device. Certain biofeedback monitoring devices may additionally or alternatively use electrodes to sense electrical resistance (e.g., a galvanic skin response) or magnetic fields (e.g., a Magnetoencephalogram, or MEG), while other electrodes may be used for neuro-vascular coupling (e.g., a functional near-infrared spectroscopy, or fNIR) to sense blood flow. To effectively interpret the biofeedback signal readings at the biofeedback monitoring devices, the contact between the electrode and the body should be maintained.

Multiple types of electrode contacts for electrode-to-skin contact are available, including dry contacts (i.e., direct contact with skin) and wet contacts (i.e., in contact with the skin via a conductive medium). In some applications, a biofeedback monitoring device may use a single electrode to measure biofeedback signals, while in other applications a variety of multi-electrode arrangements may be used for receiving biofeedback signals at the biofeedback monitoring device. In one such multi-electrode example, a multiple electrode arrangement may be coupled via a pigtail of wires to the biofeedback monitoring device. In another multi-electrode example, multiple electrode pins may be coupled to a housing, such as in a wire brush or a pin grid arrangement. An EEG device may use a pin grid electrode contact arrangement, for example, to sense biofeedback signals of a user undergoing the tests on regions of the user's head with hair. Under such conditions where the user's hair may compromise the contact between an electrode and the user's head, not all of the electrodes of a multi-electrode arrangement may be in contact with the skin.

Knowing whether a particular electrode is making proper contact presents a particular challenge, as it may not be obvious whether an electrode has a lost or otherwise compromised contact. For example, certain biofeedback monitoring devices used in applications wherein the user is moving during everyday activities, or wherein the user is an athlete training, the electrode contact may be lost, or intermittent, potentially interfering with the results of the measured biofeedback signals, Further, because certain organs emit biofeedback signals in different frequency ranges, a compromised electrode contact may be difficult to detect. For example, a compromised electrode contact may be masked due to EEG signals coexisting with ECG and EMG signals, which are about two to three orders of magnitude larger than the EEG signals. As a result, when attenuated ECG and/or EMG signals are combined with receiver noise, the resulting signal may appear as though an EEG signal is present. Such a compromised electrode contact going undetected over any period of time could provide false readings and possibly result in an incorrect medical diagnosis.

From US 2014/296682 A1 a biological-information acquisition apparatus is known that includes a plurality of flexible attachment devices each provided with an electrode that is attached to a body and that is configured to acquire biological information, and a connector configured to connect the plurality of attachment devices.

In SEULKI LEE ET AL: "Low Power and Self-Reconfigurable WBAN Controller for Continuous Bio-Signal Monitoring System", IEEE TRANSACTIONS ON BIOMEDICAL CIRCUITS AND SYSTEMS, IEEE, US, vol. 7, no. 2, pages 178-185, a WBAN controller with Branched Bus (BB) topology and Continuous Data Transmission (CDT) protocol with low power consumption and self-reconfigurability is proposed for wearable healthcare applications. The BB topology and CDT protocol is a combination of conventional Bus and Star topology and a variation from TDMA protocol, respectively, while they are able to compensate for the electrical fault in bio-signal monitoring system caused by the electrode deformation. Thanks to them, the proposed WBAN controller enables more reliable operation in continuous bio-signal monitoring applications such as sleep monitoring.

US 2010/315206 A1 relates to coupler device, processing apparatus and method of processing a plurality of body-coupled communication signals which have been detected by using an electrode arrangement with a plurality of electrodes or electrode segments. Respective transmission parameters of the body-coupled communication signals are estimated and at least one of a selecting and weighting processing is applied to the detected body-coupled communication signals based on the estimated transmission parameters. Then, the processed body-coupled communication signals are combined to generate a diversity output signal.

US 2011/306469 A1 relates to an exercise system for giving feedback to a user thereof and to a method for communication between different devices in the exercise system. The exercise system comprises at least two body coupled communication modules for forming a body area network and at least one exercise device comprising at least one sensor, feedback means and one of the at least two body coupled communication devices. The exercise system further comprises a processing unit for collecting and processing user data from the at least one sensor and output the processed data on the feedback means via the body area network in order to give the user feedback on his exercise.

WO 2014/027298 A1 relates to a neurofeedback system that comprises an electrode for contacting skin of a user for measuring a biofeedback signal of the user, a first signal processing unit for determining a signal characteristic of the measured biofeedback signal, wherein the signal characteristic represents a neurofeedback, a second signal processing unit for determining a biofeedback signal quality of the measured biofeedback signal by extracting a signal feature of the measured biofeedback signal and calculating a probability of a measurement error for said signal feature, which probability represents the biofeedback signal quality, and a feedback unit for providing feedback to the user, wherein the feedback comprises the neurofeedback and a feedback about the biofeedback signal quality. A further aspect relates to a method for supporting a behavior change of a person and a computer program for carrying out said method.

### BRIEF DESCRIPTION OF THE DRAWINGS

The concepts described herein are illustrated by way of example and not by way of limitation in the accompanying figures. For simplicity and clarity of illustration, elements illustrated in the figures are not necessarily drawn to scale. Where considered appropriate, reference labels have been repeated among the figures to indicate corresponding or analogous elements.
FIG. 1 is a simplified illustration of at least one embodiment of an electrode contact analysis system including a biofeedback monitoring device in communication with a contact analysis device;
FIG. 2 is a simplified illustration of at least one other embodiment of an electrode contact analysis system including a biofeedback monitoring device that includes a contact analysis device;
FIG. 3 is a simplified block illustration of at least one embodiment of an electrode arrangement;
FIG. 4 is a simplified illustration of at least one embodiment of an electrode arrangement in a pin grid arrangement;
FIG. 5 is a simplified block diagram of at least one embodiment of an environment of the electrode contact analysis system of FIG. 1;
FIG. 6 is a simplified block diagram of at least one embodiment of an environment of the electrode contact analysis system of FIG. 2;
FIG. 7 is a simplified flow diagram of at least one embodiment of a method for monitoring electrode contact that may be executed by the electrode contact analysis system of FIGS. 1 and 2; and
FIG. 8 is a simplified flow diagram of at least one embodiment of another method for monitoring electrode contact that may be executed by the electrode contact analysis system of FIGS. 1 and 2 using a BCC reference signal identifier.

### DETAILED DESCRIPTION OF THE DRAWINGS

While the concepts of the present disclosure are susceptible to various modifications and alternative forms, specific embodiments thereof have been shown by way of example in the drawings and will be described herein in detail. It should be understood, however, that there is no intent to limit the concepts of the present disclosure to the particular forms disclosed, but on the contrary, the intention is to cover all modifications, equivalents, and alternatives consistent with the present disclosure and the appended claims.

The invention is defined by the claims. References in the specification to "one embodiment," "an embodiment," "an illustrative embodiment," etc., indicate that the disclosure may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. Additionally, it should be appreciated that items included in a list in the form of "at least one of A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on one or more transitory or non-transitory machine-readable (e.g., computer-readable) storage media, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

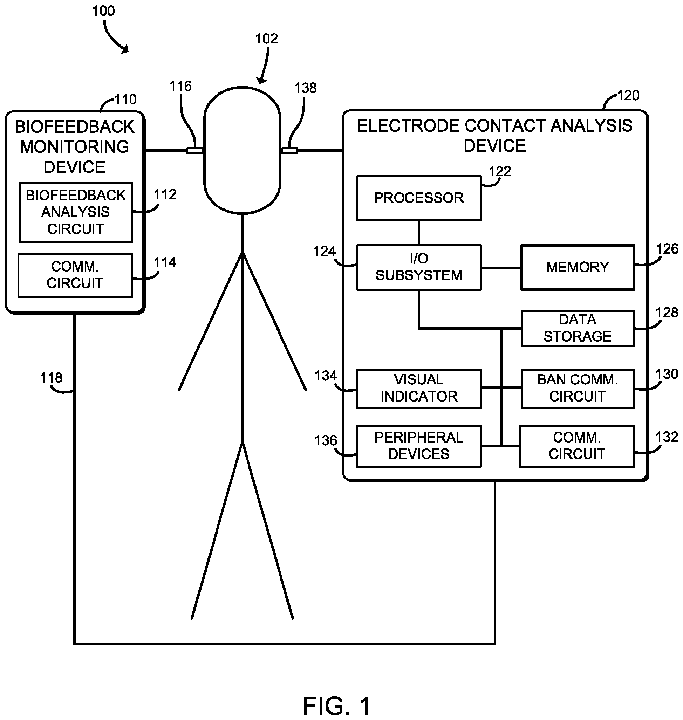

Referring now to FIG. 1, an illustrative embodiment of an electrode contact analysis system 100 includes a biofeedback monitoring device 110 and an electrode contact analysis device 120. The illustrative system 100 is shown in use in FIG. 1, and each of the biofeedback monitoring device 110 and the electrode contact analysis device 120 are in contact with a user 102 via corresponding electrodes (i.e., electrodes 116 and 138, respectively). The electrodes 116, 138 may be in direct contact with skin of the body of the user 102 (i.e., a dry contact), or in contact with a conductive medium applied to the skin of the body of the user 102 (i.e., a wet contact interface), such as an electrically conductive gel. The electrodes 116, 138 may be embodied as any type of electrical conductor capable of transmitting and/or receiving an electric current.

It should be appreciated that, in a typical biofeedback monitoring device, a loss of contact between the electrode 116 and the user 102 may not be obvious or otherwise difficult to detect. For example, in a situation in which an attenuated biofeedback ECG or EMG signal in the 100 mV range is picked up by the electrode 116 detecting a biofeedback EEG signal whose contact has been compromised (i.e., a loss of contact between the electrode 116 and the user 102) and combined with receiver noise, it may appear to the biofeedback monitoring device 110 as though a biofeedback EEG signal in the 50-100 µV is still present. However, in the presented embodiments discussed in further detail below, the electrode contact analysis device 120 is configured to determine a quality of contact between the electrode 116 and a body of the user 102. To do so, the electrode contact analysis device 120 transmits a body coupled communication (BCC) reference signal through the user 102 via a transmission electrode (e.g., electrode 138), which is received by the biofeedback monitoring device 110 as part of a biofeedback signal.

As discussed above, the biofeedback monitoring device 110 is configured to receive biofeedback signals, such as an electrical activity signal of an organ of the body of the user 102 (as well as any BCC reference signal injected by the electrode contact analysis device 120), via a receive electrode (e.g., the electrode 116). Subsequent to receiving the biofeedback signals, the biofeedback monitoring device 110 may perform any signal analysis typically performed by the biofeedback monitoring device 110 (e.g., an ECG signal analysis, an EMG signal analysis, a EEG signal analysis, etc.). Additionally, in the illustrative embodiment of FIG. 1, the biofeedback monitoring device 110 transmits the biofeedback signals to the electrode contact analysis device 120 via a communication connection 118. As discussed in more detail below, the electrode contact analysis device 120 processes the biofeedback signals received from the biofeedback monitoring device 110 to produce a received BCC reference signal from the received biofeedback signals. The received BCC reference signal is used to determine the contact quality of the electrode 116 of the biofeedback monitoring device 110. In some embodiments, the contact quality may be determined based on a comparison of the transmitted BCC reference signal and the transmitted reference signal, such as by comparing a level of attenuation between the two signals. The contact quality may then be provided to an operator of the electrode contact analysis system 100. In this way, when the contact quality of the electrode 116 is compromised, the operator can identify the electrode 116 that is presently compromised, correct the contact, and/or calibrate the electrode 116 that is presently compromised.

The biofeedback monitoring device 110 may be embodied as any type of biofeedback monitoring device capable of performing the functions described herein. For example, the biofeedback monitoring device 110 may be embodied as, or otherwise include, an electroencephalogram (EEG) monitoring device, an electrocardiogram (ECG) monitoring devices, an electromyogram (EMG) monitoring device, and/or any other biofeedback monitoring device that utilizes one or more electrodes to monitor for biofeedback signals of a user. The illustrative biofeedback monitoring device 110 includes a biofeedback analysis circuit 112 and a communication circuit 114. Of course, the biofeedback monitoring device 110 may include additional and/or alternative features and/or components based on type of biofeedback to be monitored and/or its intended use in other embodiments.

The biofeedback analysis circuit 112 may be embodied as any type of circuit capable of receiving and analyzing biofeedback signals of the user 102 via the electrode 116. The particular components, devices, and/or sub-circuits of the biofeedback analysis circuit 112 may depend on the type of biofeedback signal (e.g., EEG, ECG, EMG, etc.) the biofeedback monitoring device 110 is configured to analyze. As such, the biofeedback analysis circuit 112 may be embodied as, or otherwise be similar to, a biofeedback analysis circuit found in a typical biofeedback monitoring device. In use, the biofeedback analysis circuit 112 is configured to provide any received biofeedback signals to the communication circuit 114, in addition to performing any intended analysis of the biofeedback signals.

The communication circuit 114 is configured to communicate with the electrode contact analysis device 120 via the communication connection 118. The communication circuit 114 of the electrode contact analysis device 120 may be embodied as any communication circuit capable of enabling communications between the biofeedback monitoring device 110 and the electrode contact analysis device 120. Depending on the particular type of communication modalities supported by biofeedback monitoring device 110 and the electrode contact analysis device 120, the communication circuit 114 may be embodied as, or otherwise include, a cellular communication circuit, a data communication circuit, and/or other communication circuit technologies. As such, the communication circuit 114 may be configured to use any one or more suitable communication technology (*e.g*., wireless or wired communications) and associated protocols (*e.g.,* GSM, CDMA, FireWire, RS232, Ethernet, USB, Bluetooth®, ZigBee®, Wi-Fi®, WiMAX, etc.) to effect such communication. Similarly, the communication connection 118 may be embodied as a wired and/or wireless communication connection depending on the communication modality used by the communication circuit 114.

The electrode contact analysis device 120 may be embodied as any type of device, circuit, or collection thereof capable of determining a contact quality of a contact of the biofeedback monitoring device 110 and performing the functions described herein. The illustrative electrode contact analysis device 120 includes a processor 122, an input/output (I/O) subsystem 124, a memory 126, a data storage 128, a body area network (BAN) communication circuit 130, a communication circuit 132, a visual indicator 134, and peripheral devices 136. Of course, it should be appreciated that, in some embodiments, additional and/or alternative features and/or components may be included in the electrode contact analysis device 120.

The processor 122 may be embodied as any type of processor capable of performing the functions described herein. For example, the processor 122 may be embodied as a single or multi-core processor(s), digital signal processor, microcontroller, or other processor or processing/controlling circuit. Similarly, the memory 126 may be embodied as any type of volatile or non-volatile memory or data storage capable of performing the functions described herein. In operation, the memory 126 may store various data and software used during operation of the electrode contact analysis device 120 such as operating systems, applications, programs, libraries, and drivers. The memory 126 is communicatively coupled to the processor 122 via the I/O subsystem 124, which may be embodied as circuitry and/or components to facilitate input/output operations with the processor 122, the memory 126, and other components of the electrode contact analysis device 120. For example, the I/O subsystem 124 may be embodied as, or otherwise include, memory controller hubs, input/output control hubs, firmware devices, communication links (*i.e.,* point-to-point links, bus links, wires, cables, light guides, printed circuit board traces, etc.) and/or other components and subsystems to facilitate the input/output operations. In some embodiments, the I/O subsystem 124 may form a portion of a system-on-a-chip (SoC) and be incorporated, along with the processor 122, the memory 126, and other components of the electrode contact analysis device 120, on a single integrated circuit chip.

Similarly to the communication circuit 114 of the biofeedback monitoring device 110, the communication circuit 132 of the electrode contact analysis device 120 may be embodied as any communication circuit capable of enabling communications between the electrode contact analysis device 120 and the biofeedback monitoring device 110. Depending on the particular type of communication modalities supported by electrode contact analysis device 120 and the biofeedback monitoring device 110, the communication circuit 132 of the electrode contact analysis device 120 may be embodied as, or otherwise include, a cellular communication circuit, a data communication circuit, and/or other communication circuit technologies. The communication circuit 132 may be configured to use any one or more suitable communication technology (*e.g*., wireless or wired communications) and associated protocols (*e.g.,* GSM, CDMA, FireWire, RS232, Ethernet, USB, Bluetooth®, ZigBee®, Wi-Fi®, WiMAX, etc.) to effect such communication. In some embodiments, the communication circuit 114 and/or the communication circuit 132 may be further configured to transmit and/or receive wired and/or wireless communications with a remote external device, such as a remote network device (e.g., an access point) and/or a remote computing device (e.g., a workstation).

The data storage 128 may be embodied as any type of device or devices configured for short-term or long-term storage of data such as, for example, memory devices and circuits, memory cards, hard disk drives, solid-state drives, or other data storage devices. Additionally, the electrode contact analysis device 120 may store the various communication protocols and BCC reference signal mapping schemes in the data storage 128 which are used to generate and interpret the BCC reference signals as discussed in more detail below.

The BAN communication circuit 130 may be embodied as any communication circuit, device, or collection thereof, capable of transmitting and receiving BAN signals between the electrode contact analysis device 120 and the body of the user 102 via the electrode 138. As will be described in further detail below, the BAN communication circuit 130 generates the BCC reference signal in use.

The visual indicator 134 of the contact may be embodied as any type of visual indicator capable of displaying a visual indication to the user 102 or the operator of the electrode contact analysis device 120. In some embodiments, the visual indicator 134 may be embodied as any type of display capable of displaying digital information such as a liquid crystal display (LCD), a plasma display, a cathode ray tube (CRT), or other type of display device. In such embodiments, the display may be embodied as a touch screen (e.g., a resistive touch screen, a capacitive touch screen, or the like) capable of generating input data in response to being touched by the user 102 and/or the operator of the electrode contact analysis device 120. In some embodiments, the visual indicator 134 may be embodied as one or more lights (e.g., light emitting diode, or LEDs) capable of providing an indication of a quality of a contact between the electrode 116 of the biofeedback monitoring device 110 and the user 102. In such embodiments, the light(s) may be capable of providing a variety of colors, luminance levels, and/or flash patterns to provide the indication of the quality of the contact between the electrode 116 and the user 102. While the illustrative embodiment includes a visual indicator 134, it is contemplated herein that any type of feedback indicator, such as an audio indicator (i.e., speaker), printer, etc. may be used additionally or alternatively to the visual indicator 134. In embodiments using an audible indicator, the presence of an audible tone (e.g., a continuity check) may indicate the quality of the contact between the electrode 116 and the user 102 is at or above an acceptable threshold of contact. In other embodiments using an audible indicator, the audible tone may be embodied as a sequence of beeps or emitted at a particular volume corresponding to the quality of the contact between the electrode 116 and the user 102 being at or above an acceptable threshold of contact.

Additionally, in some embodiments, the electrode contact analysis device 120 may further include one or more peripheral devices 136. Such peripheral devices 136 may include any type of peripheral device commonly found in a computing device, and particularly a biofeedback monitoring device, such as a hardware keyboard, input/output devices, peripheral communication devices, and/or the like, for example.

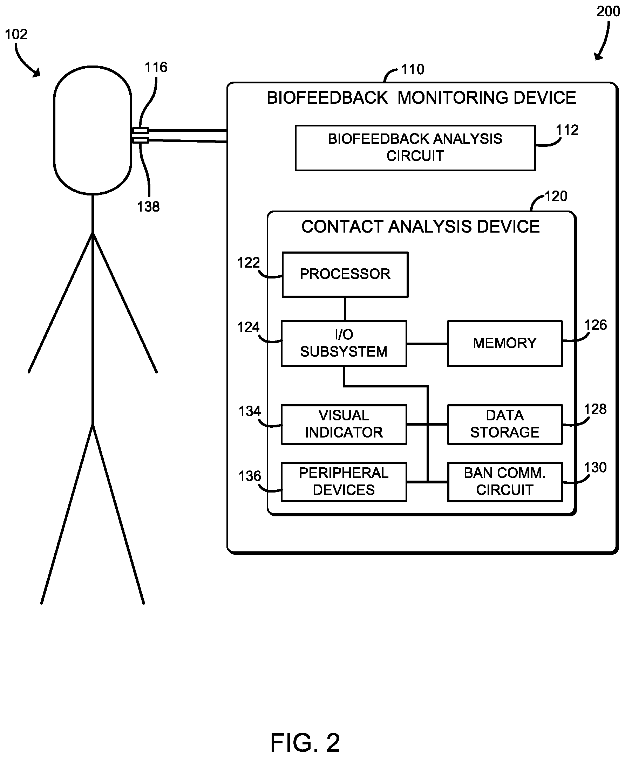

In some embodiments, the electrode contact analysis device 120 may be integrated into the biofeedback monitoring device 110 as shown in the illustrative electrode contact analysis system 200 of FIG. 2. Similar to the electrode contact analysis system 100 illustrated in FIG. 1, the biofeedback monitoring device 110 of the electrode contact analysis system 200 includes the same components with the exception of the communication circuit 114 of the biofeedback monitoring device 110 and the communication circuit 132 of the electrode contact analysis device 120. As such, further descriptions of the like components are not repeated herein for clarity of the description with the understanding that the description of the corresponding components provided above in regard to the electrode contact analysis system 100 of FIG. 1 applies equally to the corresponding components of the electrode contact analysis system 200 of FIG. 2. Of course, a communication circuit may be included in the electrode contact analysis system 200 in some embodiments. In such embodiments, the communication circuit may allow the electrode contact analysis device 120 and/or the biofeedback monitoring device 110 to communicate with an external device, such as a network device (e.g., an access point) and/or a computing device (e.g., a workstation), via wired and/or wireless communication technologies. Additionally, in some embodiments, certain components illustrated as being located exclusively in the electrode contact analysis device 120 or the biofeedback monitoring device 110 may be located in one device 110 or 120 and shared between both devices 110 and 120.

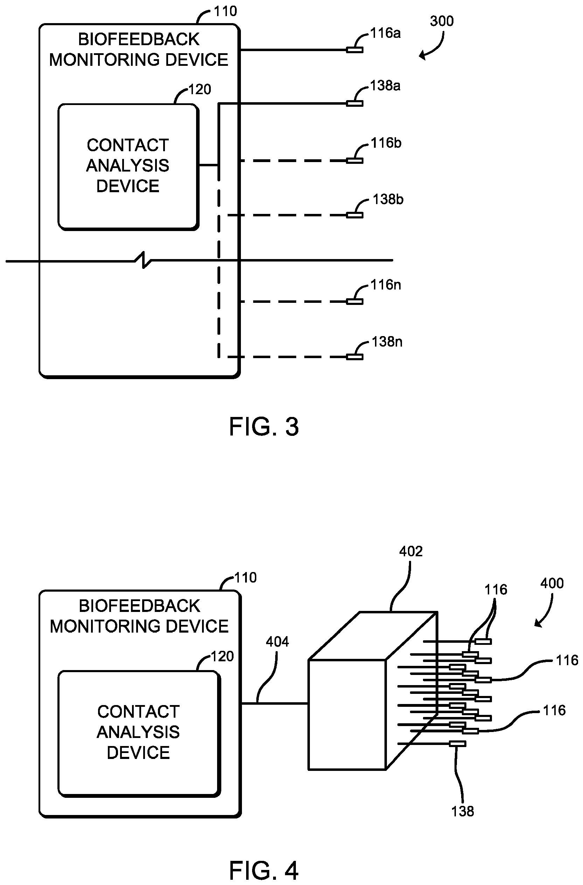

The electrode contact analysis systems 100, 200 may utilize various types of electrode arrangements for sensing biofeedback signals including, for example, individual electrode pins or pads, electrode pin blocks, and/or other arrangements. One illustrative embodiment of an electrode arrangement 300 usable by the electrode contact analysis system 200 of FIG. 2 is shown in FIG. 3. As will be discussed in further detail below, the quality of the contact of a receive electrode 116 is determined based on a BCC reference signal transmitted from a transmission electrode 138. Depending on the implementation, an electrode arrangement may include a single receive electrode 116 and a single transmission electrode 138. In such embodiments, the system 200 may extrapolate the contact quality of other receive electrodes 116 based on the determined contact quality of the receive electrode 116 as discussed below. However, in other embodiments, multiple receive electrodes 116 (e.g., 116a, 116b, ..., 116n) and/or multiple transmission electrodes 138 (e.g., 138a, 138b, ..., 138n) may be included in the electrode arrangement as discussed in more detail below.

In embodiments in which the electrode arrangement 300 includes multiple transmission electrodes 138, each transmission electrode 138 may transmit a corresponding (and possibly different) BCC reference signal, which is received by a single receive electrode 116. In an example of such an embodiment, the contact quality of electrode 116a may be based at least in part on BCC reference signals transmitted from electrodes 138a and 138b (see FIG. 3). Alternatively, the electrode arrangement 300 may include a single transmission electrode 138 to transmit a BCC reference signal and more than one receive electrode 116 to receive biofeedback signals. In an example of such an embodiment, the contact quality of receive electrodes 116a and 116b may be based at least in part on a BCC reference signal transmitted from electrode 138a (see FIG. 3). In other embodiments, the electrode arrangement 300 may include more than one transmission electrode 138 to transmit a corresponding number of BCC reference signals (which may be different BCC reference signals) and more than one receive electrode 116 to receive biofeedback signals. In an example of such an embodiment, the contact quality of receive electrode 116a may be based at least in part on the BCC reference signal transmitted from transmission electrode 138a, while the contact of receive electrode 116b may be based at least in part on the BCC reference signal transmitted from transmission electrode 138b, and so forth. Of course, other combinations of receive electrodes 116 and/or transmission electrode 138 may be implemented in an electrode arrangement of the systems 100, 200. The specific example embodiments provided herein are intended to illustrate, and should not be construed as limitations of the electrode arrangement 300.

Referring now to FIG. 4, in some embodiments, the electrodes of the systems 100, 200 may be embodied as a pin block or pin grid arrangement 400. The pin grid arrangement 400 includes a housing 402 from which the receive and transmission electrodes 116, 138 extend. In the illustrative embodiment, the housing 402 is connected to the biofeedback monitoring device 110 by a communication channel 404. The communication channel 404 may be embodied as any wired and/or wireless communication technology capable of transmitting and receiving data (i.e., signals) between the biofeedback monitoring device 110 and the electrodes 116, 138 of the pin grid arrangement 400. In the illustrative embodiment, a single transmission electrode 138 is designated to transmit a BCC reference signal, and the remaining electrodes 116 are designated to receive biofeedback signals. In order to preserve clarity, only a few of the electrodes 116 have been specifically designated by reference numerals in the illustrated embodiment of FIG. 4. It should be appreciated that while electrode 138 is the only electrode designated to transmit a BCC reference signal in the illustrative embodiment of the pin grid arrangement 400, other electrode pin designations as described above may be present in other embodiments. For example, the pin grid arrangement 400 may include multiple transmission electrodes 138.

In some embodiments, an individual electrode may be configured to perform both the function of the transmit signal electrode 138 and the receive signal electrode 116 (e.g., via temporally multiplexing the dual functions). For example, an electrode of the pin grid arrangement 400 previously set as a receive electrode 116 may be configured to function as a transmission electrode 138 for a period of time until it is reverted back to a receive electrode 116, at which time another electrode of the pin grid arrangement 400 may be set to function as the transmit electrode 138. In other words, the electrodes of the pin grid arrangement 400 that function as the transmit electrode 138 may change over time. Additionally, in some embodiments, such as the electrode contact analysis system 100 of FIG. 1, each electrode of the pin grid arrangement may be configured to function as a receive electrode 116, while the transmit electrode 138 transmits the BCC reference signal from an external electrode contact analysis device 120. Alternatively, in embodiments in which each electrode of the pin grid arrangement 400 is configured to function as a receive electrode 116, the transmit electrode 138 may transmit the BCC reference signal from another electrode contact analysis system, such as the electrode contact analysis system 200.

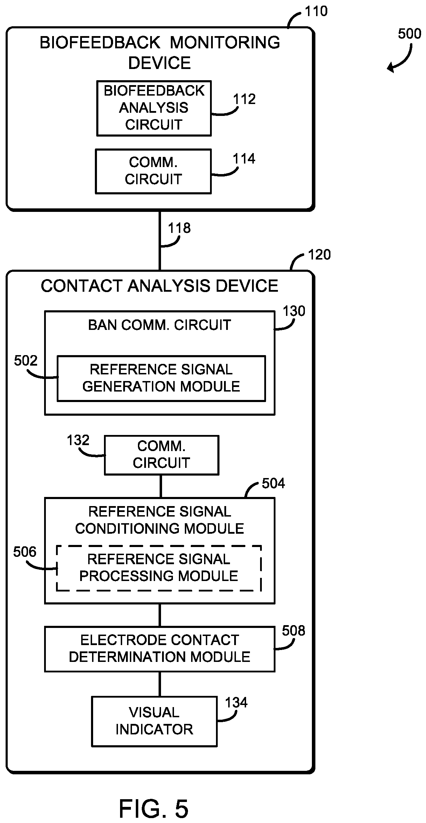

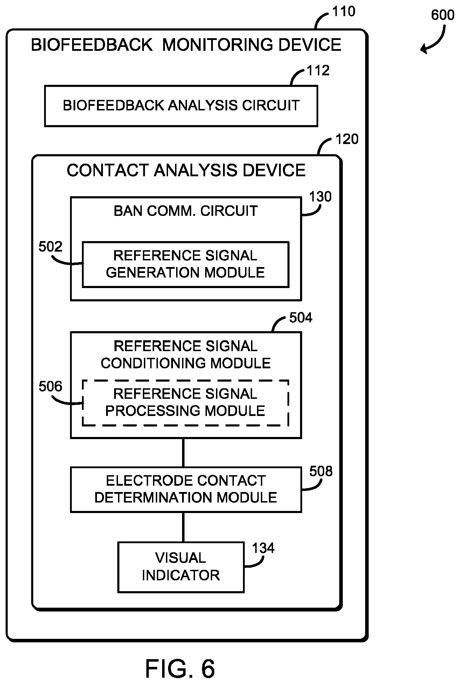

With reference to FIGS. 5 and 6, illustrative embodiments of environments 500 and 600 established during operation of the electrode contact analysis systems 100, 200, respectively, are shown. Specifically, the electrode contact analysis system 100 of FIG. 1 may establish the environment 500, illustrated in FIG. 5, in which the electrode contact analysis device 120 is coupled externally to the biofeedback monitoring device 110. Similarly, the electrode contact analysis system 200 of FIG. 2 may establish the environment 600, illustrated in FIG. 6, in which the electrode contact analysis device 120 is integrally coupled to the biofeedback monitoring device 110. As discussed below, the environments 500, 600 generally include similar modules and functionality.

Referring to FIG. 5, in the illustrative embodiment, the electrode contact analysis system 100 of FIG. 1 establishes an environment 500 during operation. The environment 500 includes a reference signal generation module 502, a reference signal conditioning module 504, and an electrode contact determination module 508. The various modules of the environment 500 may be embodied as hardware, firmware, software, or a combination thereof. For example, each of the modules, logic, and other components of the environment 500 may form a portion of, or otherwise be established by, the processor 122 or other hardware components of the electrode contact analysis device 120. For example, in some embodiments, one or more of the modules of environment 500 may be embodied as a circuit (e.g., a reference signal generation circuit, a reference signal condition circuit, and/or an electrode contact determination circuit).

The reference signal generation module 502 is configured to generate a BCC reference signal, which is output to the electrode 138 for conducting the BCC reference signal to the body of the user 102. In some embodiments, the electrode 138 may conduct the BCC reference signal to the body of the user 102 by capacitive coupled signaling or galvanic coupled signaling. As will be described in further detail below, in some embodiments, the BCC reference signal may be a stand-alone signal or a signal modulated with data. In such an embodiment in which the BCC reference signal is modulated with data, the data may be embodied as a unique identifier corresponding to the electrode 138, the electrode contact analysis device 120, and/or the biofeedback monitoring device 110. In some embodiments, such as those embodiments including more than one electrode 138, the reference signal generation module 502 may be configured to generate a different BCC reference signal for each electrode 138.

The reference signal conditioning module 504 is configured to condition the biofeedback signals sensed by the electrode(s) 116 of the biofeedback monitoring device 110 to accurately measure the signal. The reference signal conditioning module 504 may use any useful conditioning on the biofeedback signal received at the electrode contact analysis device 120. For example, the biofeedback signals received at the reference signal conditioning module 504 from the electrode(s) 116 of the biofeedback monitoring device 110 may be amplified, filtered, isolated, excited, quantized, linearized, converted, or otherwise adapted by the reference signal conditioning module 504 to recover or otherwise obtain a BCC reference signal from the biofeedback signals. In some embodiments, reference signal conditioning module 504 may include a reference signal processing module 506 to process the conditioned BCC reference signal. The reference signal processing module 506 may convert or otherwise adapt the conditioned BCC reference signal to data indicative of, or otherwise based on, the conditioned BCC reference signal (e.g., a digitized representation of the conditioned BCC reference signal). In certain embodiments, the reference signal processing module 506 may demodulate the conditioned BCC reference signal to recover additional data, such as a unique identifier associated with a source of the BCC reference signal.

The electrode contact determination module 508 is configured to determine a quality of a contact between the electrode 116 and the body of the user 102 based on the recovered BCC reference signal. In some embodiments, the quality of the contact between the electrode 116 and the user 102 is based on the conditioned BCC reference signal and/or the data indicative of the conditioned BCC reference signal. The electrode contact determination module 508 is further configured to provide the quality of the contact between the electrode 116 and the user 102 to the visual indicator 134. The visual indicator receives and interprets the contact quality of the electrode 116 and provides a visual feedback to the user 102 corresponding to the contact quality of the electrode 116. In some embodiments, the electrode contact determination module 508 may be further configured to determine whether the recovered BCC reference signal corresponds to the electrode 116 having received the biofeedback signals. For example, if the recovered BCC reference signal includes a unique identifier, the electrode contact determination module 508 may perform a lookup, such as via a lookup table, for example, to determine whether the recovered BCC reference signal is to be used to determine the contact quality of the electrode 116.

Referring now to FIG. 6, in the illustrative embodiment, the electrode contact analysis system 200 of FIG. 6 establishes an environment 600 during operation. Similarly to the illustrative embodiment of the environment 500, the illustrative environment 600 includes the reference signal generation module 502, the reference signal conditioning module 504, and the electrode contact determination module 508. However, as shown in FIG. 6, the electrode contact analysis device 120, and the modules generated thereby, are incorporated into the biofeedback monitoring device 110. Again, the various modules of the environment 600 may be embodied as hardware, firmware, software, or a combination thereof. For example, each of the modules, logic, and other components of the environment 600 may form a portion of, or otherwise be established by, the processor 122 or other hardware components of the electrode contact analysis device 120. As such, further descriptions of the like components are not repeated herein for clarity of the description with the understanding that the description of the corresponding components provided above in regard to the environment 500 of FIG. 5 applies equally to the corresponding components of the environment 600 of FIG. 6.

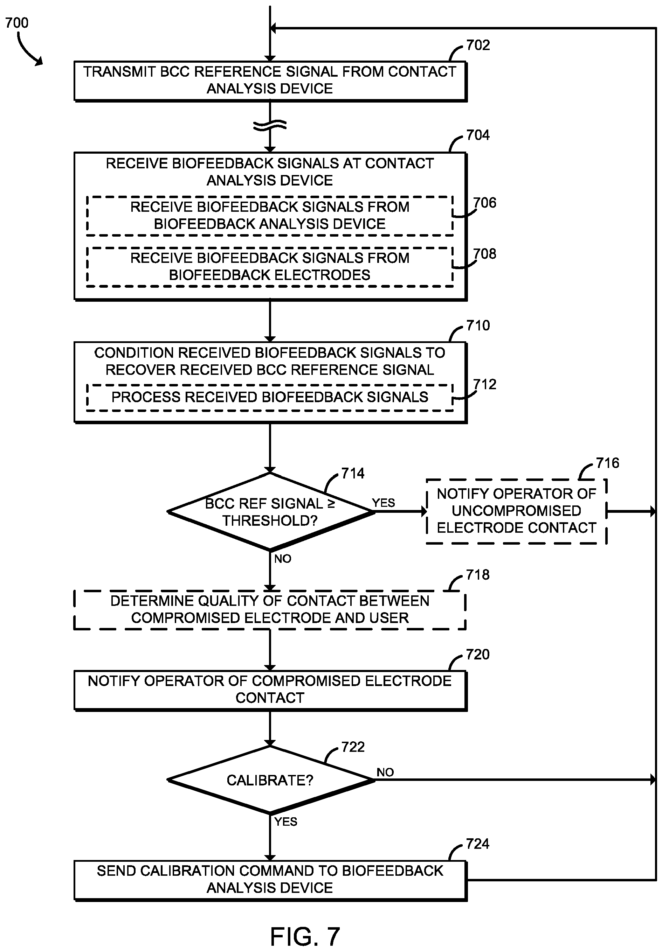

Referring now to FIG. 7, in embodiments in which a single BCC reference signal is used (e.g., either via a signal transmission electrode 138 or multiple transmission electrodes 138 transmitting the same BCC reference signal), the electrode contact analysis device 120 may execute a method 700 for monitoring the quality of contact between an electrode and an associated user. The method 700 begins with block 702, in which the electrode contact analysis device 120 transmits a generated BCC reference signal via a transmission electrode 138. As noted above, the BCC reference signal may be transmitted via a capacitive coupled transmission and/or a galvanic coupled transmission. Galvanic coupling generally provides a more direct response to attenuation in a connection of the electrode 138 to the skin, and is typically less impacted by changes in environment around the user 102. Galvanic coupling may incur a larger attenuation of the BCC reference signal than using capacitive coupled signaling; however, in an EEG application, for example, the electrode 116 is typically designed to detect small magnitude signals (e.g., 10s of microvolts), and distances associated with a head of the user 102 are generally relatively small. Capacitive coupling may be subject to changes in the environment of the user 102, particularly changes in the environment occurring in close proximity to the user 102, which may need to be compensated for. A common mode change in the recovered BCC reference signal at the electrode 116 may be used to compensate for the changes by subtracting the change from the biofeedback signals received at the electrode 116. Subtracting the change from the biofeedback signals received at the electrode 116 to compensate for the common mode change may provide a more accurate representation of the received reference signal. In some embodiments, the compensation for a change of the environment at the electrode of the biofeedback monitoring device is compensated for as a function of time. Capacitive coupling, unlike galvanic coupling, does not require direct contact to the skin of the user 102, which allows for more flexibility in coupling the electrode 138 to the body of the user 102 and potentially reduces reliability issues associated with the indirect contact between the electrode 138 and the body of the user 102. While capacitive coupling and galvanic coupling have been described herein, it should be understood that any type of signal coupling technology capable of transmitting the BCC reference signal via the electrode 138 is contemplated herein.

Subsequently, in block 704, the electrode contact analysis device 120 receives the biofeedback signals detected by a receive electrode 116 of the biofeedback monitoring device 110. As noted above, the biofeedback signals may be transmitted to the electrode contact analysis device 120 via an external communication connection 118 between the communication circuits 114 and 132 as illustrated in FIGS. 1 and 5, and indicated in block 706, or internally (i.e., from the electrode 116 through internal circuitry) as illustrated in FIG. 2 and 6, and indicated in block 708.

In block 710, the electrode contact analysis device 120 conditions the biofeedback signals received from the biofeedback monitoring device 110. As described above, the biofeedback signals received from the electrode(s) 116 of the biofeedback monitoring device 110 may be amplified, filtered, isolated, excited, quantized, linearized, converted, or otherwise adapted to recover a BCC reference signal from the received biofeedback signals. In some embodiments, in block 712 the electrode contact analysis device 120 may further process the recovered BCC reference signal. For example, the electrode contact analysis device 120 may perform an analog to digital conversion of the recovered BCC reference signal to determine a digital representation of the recovered BCC reference signal such that the digital representation can be quantified and used to compare against the threshold.

In block 714, the electrode contact analysis device 120 determines whether the recovered BCC reference signal is greater than or equal to a threshold. The threshold may be embodied as a threshold signal based on the transmitted BCC reference signal to provide a base for determining whether the contact between the electrode 116 of the biofeedback monitoring device 110 and the body of the user 102 has been compromised. The threshold may additionally or alternatively be a numerical value threshold to provide a base value for comparing the digital representation of the recovered BCC reference signal to determine whether the contact between the electrode 116 of the biofeedback monitoring device 110 and the body of the user 102 has been compromised and/or a contact quality of the electrode 116. For example, the threshold may be embodied as a signal amplitude or magnitude to which the BCC reference signal is compared. In such embodiments, the quality of the contact of the electrode(s) 116 may be inferred based on the attenuation (i.e., reduction of signal strength) of the BCC reference signal as determined by the comparison to the threshold.

If the recovered BCC reference signal is greater than or equal to the threshold, the method 700 loops back to block 702. In some embodiments, prior to the method 700 looping back to block 702, the electrode contact analysis device 120 may notify the operator and/or the user 102 that the contact between the electrode 116 and the user 102 is not compromised in block 716. In other words, a feedback via the visual indicator 134 may be used to provide the operator and/or the user 102 with an indication that the electrode 116 is within an acceptable degree of contact with the user 102.

If the recovered BCC reference signal is greater than or equal to the threshold, the method 700 continues to block 720, in which the electrode contact analysis device 120 notifies the operator and/or the user 102 that the contact between the electrode 116 and the user is compromised. In other words, a feedback via the visual indicator 134 may be used to provide the operator and/or the user 102 with an indication that the electrode 116 is not within an acceptable degree of contact with the user 102. As noted above, any type of visual indicator capable of displaying a visual indication of the quality of contact between the electrode(s) 116 to the user 102 and/or the operator of the electrode contact analysis device 120 is contemplated herein.

In some embodiments, in block 718, prior to notifying the operator and/or user 102 of the compromised electrode 116, the electrode contact analysis device 120 may determine a quality of contact between the comprised electrode 116 and the user 102 based on the recovered BCC reference signal. In some embodiments, the quality of contact may be determined based on a comparison between the threshold signal and the recovered BCC reference signal to determine a degree of difference between the threshold signal and the recovered BCC reference signal. For example, the degree of difference may be used to determine a more granular quality of contact to notify the user 102 and/or the operator of the electrode contact analysis device 120 with in block 720. Of course, additional and/or alternative comparisons may be performed on the recovered BCC reference signal and/or digital representation of the recovered BCC reference signal to determine a quality of the contact between the compromised electrode 116 and the user.

In block 722, the electrode contact analysis device 120 determines whether to calibrate the electrode(s) 116 that has been compromised. Certain conditions may contribute to the electrode 116 contact being compromised. For example, the contact between the electrode 116 and the user 102 may be obstructed by sweat, leakage of electrolyte, slow separation of electrode contact, or other such conditions that might obstruct the contact. Under such conditions, the user 102 and/or the operator of the electrode contact analysis device 120 may provide an input via the electrode contact analysis device 120 or the biofeedback monitoring device 110 to perform a calibration of the compromised electrode 116 to account for the condition. As noted above, some embodiments of the electrode contact analysis device 120 may include peripheral devices 136 such as, for example, a hardware keyboard, input/output devices, peripheral communication devices, and/or the like that are capable of receiving input from and/or providing output to the user. As also noted above, the display may be embodied as a touch capable of generating input data in response to being touched by the user 102 and/or the operator of the electrode contact analysis device 120. In such embodiments including a peripheral input device or a display capable of receiving input, the user 102 and/or the operator of the electrode contact analysis device 120 may provide an input that directs the electrode contact analysis device 120 to perform a calibration of the compromised electrode 116. If the user 102 and/or operator determines that a calibration should be performed, in block 724 a calibration command is sent via a BCC command signal from the transmit electrode 138. If not, or after the calibration command was sent, the method 700 loops back to block 702.

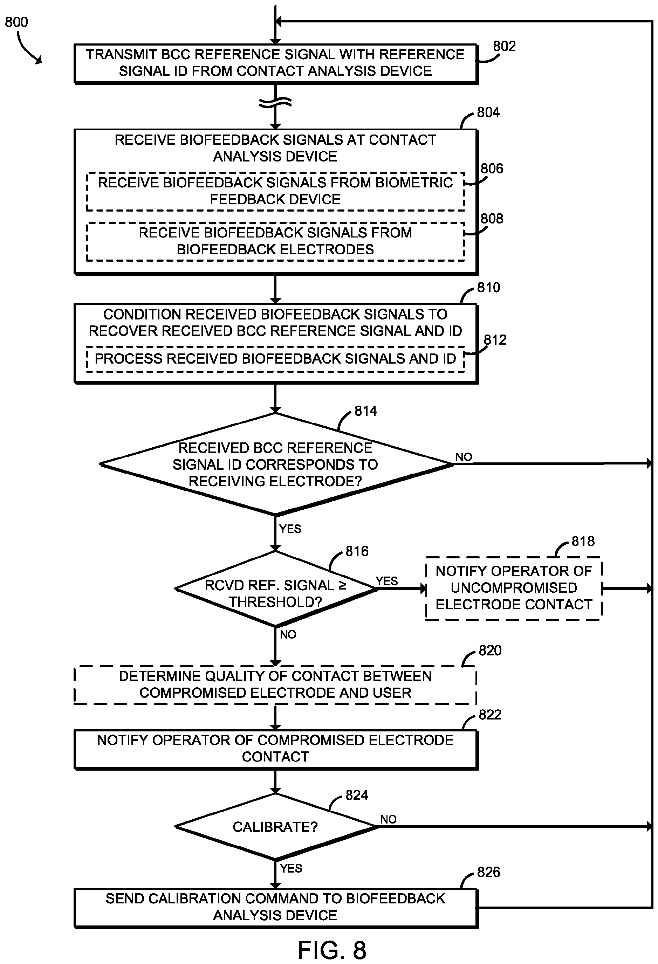

Referring now to FIG. 8, in embodiments in which multiple BCC reference signals are used (e.g., multiple transmission electrodes 138 transmitting the different BCC reference signals), the electrode contact analysis device 120 may execute a method 800 for monitoring the quality of contact between one or more electrodes and an associated user. The method 800 begins with block 802, in which the electrode contact analysis device 120 transmits a generated BCC reference signal including a reference signal identifier via the electrode 138. Similarly to block 704 of the method 700 of FIG. 7, in block 804 the electrode contact analysis device 120 receives the biofeedback signals detected by the electrode 116 of the biofeedback monitoring device 110. Similarly to blocks 706 and 708 of the method 700, the biofeedback signals may be transmitted to the electrode contact analysis device 120 via an external communication connection 118 between the communication circuits 114 and 132 as illustrated in FIGS. 1 and 5, and indicated in block 806, or internally (i.e., from the electrode 116 through internal circuitry) as illustrated in FIG. 2 and 6, and indicated in block 808.

In block 810, similarly to block 710 of the method 700, the received biofeedback signals are conditioned to recover the received BCC reference signal. Because multiple BCC reference signals are being used, each BCC reference signal may include a separate reference signal identifier. As such, additionally in block 810, the received biofeedback signals are further conditioned to recover the associated reference signal identifier transmitted via the corresponding transmission electrode 138 in block 802. In some embodiments, in block 812 the received biofeedback signals and reference signal identifier may be further processed by the electrode contact analysis device 120. As described above, the electrode contact analysis device 120 may process the received biofeedback signals and reference signal identifier by performing an analog to digital conversion of the recovered BCC reference signal and reference signal identifier to determine a digital representation of the recovered BCC reference signal and reference signal identifier.

In block 814, the electrode contact analysis device 120 determines whether the received BCC reference signal identifier corresponds to the electrode 116 that received the biofeedback signals. In some embodiments, the received BCC reference signal identifier may be compared using, for example, a lookup table to determine whether the electrode 116 that received the biofeedback signal including the BCC reference signal identifier is mapped, or assigned, to that BCC reference signal. For example, in embodiments utilizing multiple transmission electrodes 138 and multiple receive electrodes 116, as shown in FIG. 3, each pair of transmission electrodes 138 and receive electrodes 116 may be configured to use a different BCC reference signal identifier such that the receive electrodes 116 do not misconstrue a BCC reference signal transmitted by an un-paired transmission electrode 138. Additionally, in situations in which more than one electrode contact analysis device 120 and more than one biofeedback monitoring device 110 (e.g., different type of biofeedback monitoring devices) are in contact with the user 102, each biofeedback monitoring device 110 may be assigned a different BCC reference signal identifier corresponding to one of the electrode contact analysis devices 120. Under certain conditions, the biofeedback monitoring device 110 may receive biofeedback signals including a BCC reference signal that does not correspond to the electrode contact analysis device 120 assigned to the biofeedback monitoring device 110. Such a condition may result in inaccurate results. For example, where a user 102 has more than one transmit electrode 138 and more than one receive electrode 116 in contact with the user 102, each receive electrode 116 may be assigned a BCC reference signal identifier corresponding to one of the transmit electrodes 138. Under certain conditions, a receive electrode 116 may receive biofeedback signals including a BCC reference signal that does not correspond to the transmit electrode 138 assigned to the receive electrode 116. Such a condition may also result in inaccurate results.

Similarly to the method 700 illustrated in FIG. 7, blocks 816 to 826 of the method 800 perform the same functions as described above with regard to blocks 714 to 724. As such, further descriptions of the like blocks are not repeated herein for clarity of the description with the understanding that the description of the corresponding blocks provided above in regard to the method 700 illustrated in FIG. 7 applies equally to the corresponding blocks of the method 800 illustrated in FIG. 8.

### EXAMPLES

Illustrative examples of the technologies disclosed herein are provided below. A disclosure of the technologies may include any one or more, and any combination of, the examples described below.

Example 1 includes an electrode contact analysis device for monitoring contact between one or more electrodes of a biofeedback monitoring device and a user, the electrode contact analysis device comprising a body area network (BAN) communication circuit to transmit a body coupled communication (BCC) reference signal via a transmission electrode; a communication circuit to receive biofeedback signals from the biofeedback monitoring device, wherein the biofeedback signals comprise biofeedback signals received by an electrode of the biofeedback monitoring device; a reference signal processing module to process the received biofeedback signals to produce a received BCC reference signal from the received biofeedback signals; and an electrode contact determination module to determine a quality of a contact between the electrode and the user based on the received BCC reference signal.

Example 2 includes the subject matter of Example 1, and further including a reference signal generation module to generate the BCC reference signal, wherein the BCC reference signal includes an identifier, and wherein at least a portion of the identifier corresponds to one of the transmission electrode or the electrode of the biofeedback monitoring device.

Example 3 includes the subject matter of any of Examples 1 and 2, and wherein the electrode contact determination module is to determine the quality of the contact between the electrode and the user based on a comparison of the received BCC reference signal and a threshold.

Example 4 includes the subject matter of any of Examples 1-3, and wherein the electrode contact determination module is to determine the quality of the contact between the electrode and the user based on a comparison of a magnitude of the received BCC reference signal and a magnitude threshold.

Example 5 includes the subject matter of any of Examples 1-4, and wherein the electrode contact determination module is further to generate a notification to an operator of the electrode contact analysis in response to the received BCC reference signal having a predetermined relationship with the threshold.

Example 6 includes the subject matter of any of Examples 1-5, and wherein to generate the notification comprises to activate a visual indicator of the electrode contact analysis device.

Example 7 includes the subject matter of any of Examples 1-6, and wherein the BAN communication circuit transmits the BCC reference signal via one of a capacitive coupled transmission and a galvanic coupled transmission.

Example 8 includes the subject matter of any of Examples 1-7, and wherein the electrode contact determination module is further to detect a common mode change of the received BCC reference signal and to subtract the common mode change from the received BCC reference signal to provide a compensated BCC reference signal, and wherein the electrode contact determination module is to determine the quality of the contact between the electrode of the biofeedback monitoring device and the user based on the compensated BCC reference signal.

Example 9 includes the subject matter of any of Examples 1-8, and wherein the BAN communication circuit is further to transmit a calibration command via the transmission electrode to compensate for a change of an environment condition detected by the electrode of the biofeedback monitoring device.

Example 10 includes the subject matter of any of Examples 1-9, and wherein the calibration command is to compensate for the change of the environment condition at the electrode of the biofeedback monitoring device as a function of time.

Example 11 includes the subject matter of any of Examples 1-10, and wherein the BCC reference signal transmitted via the transmission electrode comprises a non-biological signal.

Example 12 includes a biofeedback monitoring device for monitoring biofeedback signals between one or more electrodes of the biofeedback monitoring device and a user, the biofeedback monitoring device comprising a biofeedback analysis circuit to receive biofeedback signals of the user via the one or more electrodes of the biofeedback monitoring device, the one or more electrodes comprising a first electrode and a second electrode; a body area network (BAN) communication circuit to transmit a body coupled communication (BCC) reference signal via the first electrode and to receive the biofeedback signals of the user via the second electrode; a reference signal processing module to process the received biofeedback signals to produce a received BCC reference signal; and an electrode contact determination module to determine a quality of a contact between the first electrode of the biofeedback monitoring device and the user based on the received BCC reference signal.

Example 13 includes the subject matter of Example 12, and further including a reference signal generation module to generate the BCC reference signal, wherein the BCC reference signal includes an identifier, and wherein at least a portion of the identifier corresponds to one of the transmission electrode or the electrode of the biofeedback monitoring device.

Example 14 includes the subject matter of any of Examples 12 and 13, and wherein the electrode contact determination module is to determine the quality of the contact between the first electrode and the user based on a comparison of the received BCC reference signal and a threshold.

Example 15 includes the subject matter of any of Examples 12-14, and wherein the electrode contact determination module is to determine the quality of the contact between the first electrode and the user based on a comparison of a magnitude of the received BCC reference signal and a magnitude threshold.

Example 16 includes the subject matter of any of Examples 12-15, and wherein the electrode contact determination module is further to generate a notification and provide the notification to an operator of the electrode contact analysis in response to the received BCC reference signal having a predetermined relationship with the threshold.

Example 17 includes the subject matter of any of Examples 12-16, and wherein the first electrode transmits the BCC reference signal via one of a capacitive coupled transmission and a galvanic coupled transmission.

Example 18 includes the subject matter of any of Examples 12-17, and wherein the electrode contact determination module is further to detect a common mode change of the BCC reference signal received by the second electrode of the biofeedback monitoring device and to subtract the common mode change from the BCC reference signal received by the second electrode to provide a compensated BCC reference signal, and wherein the electrode contact determination module is to determine the quality of the contact between the second electrode of the biofeedback monitoring device and the user based on the compensated BCC reference signal.

Example 19 includes the subject matter of any of Examples 12-18, and wherein the BAN communication circuit is further to transmit a calibration command via the first electrode to compensate for a change of an environment condition detected by the second electrode of the biofeedback monitoring device.

Example 20 includes the subject matter of any of Examples 12-19, and wherein the calibration command is to compensate for the change of the environment condition at the second electrode of the biofeedback monitoring device as a function of time.

Example 21 includes the subject matter of any of Examples 12-20, and wherein the BCC reference signal transmitted via the first electrode comprises a non-biological signal.

Example 22 includes a method for monitoring contact between one or more electrodes of a biofeedback monitoring device and a user, the method comprising transmitting, by an electrode of an electrode contact analysis device, a body coupled communication (BCC) reference signal to a body of the user; receiving, from the biofeedback monitoring device, biofeedback signals received by an electrode of the biofeedback monitoring device; processing, by the electrode contact analysis device, the received biofeedback signals to produce a received BCC reference signal; and determining, by the electrode contact analysis device, a quality of a contact between the electrode of the biofeedback monitoring device and the user based on the received BCC reference signal.

Example 23 includes the subject matter of Example 22, and further including generating, by the electrode contact analysis device, the BCC reference signal including an identifier, wherein at least a portion of the identifier corresponds to at least one of the electrode contact analysis device and the electrode of the electrode contact analysis device.

Example 24 includes the subject matter of any of Examples 22 and 23, and wherein determining the quality of the contact between the electrode and the user comprises comparing the received BCC reference signal and a threshold.

Example 25 includes the subject matter of any of Examples 22-24, and wherein comparing the received BCC reference signal and a threshold comprises comparing a magnitude of the received BCC reference signal and a magnitude threshold.

Example 26 includes the subject matter of any of Examples 22-25, and further including generating a notification in response to the received BCC reference signal having a predetermined relationship with the threshold; and providing the notification to an operator of the electrode contact analysis.

Example 27 includes the subject matter of any of Examples 22-26, and wherein generating the notification comprises activating a visual indicator of the electrode contact analysis device.

Example 28 includes the subject matter of any of Examples 22-27, and wherein transmitting the BCC reference signal to the body of the user comprises transmitting the BCC reference signal to the body of the user via one of a capacitive coupled transmission and a galvanic coupled transmission.

Example 29 includes the subject matter of any of Examples 22-28, and further including detecting, by the electrode contact analysis device, a common mode change of the received BCC reference signal by the electrode of the biofeedback monitoring device; and subtracting, by the electrode contact analysis device, the common mode change from the received BCC reference signal to provide a compensated received BCC reference signal.

Example 30 includes the subject matter of any of Examples 22-29, and further including transmitting, by the electrode contact analysis device, a calibration command to compensate for a change of an environment condition detected by the electrode of the biofeedback monitoring device.

Example 31 includes the subject matter of any of Examples 22-30, and wherein transmitting the calibration command comprises compensating for the change of the environment condition at the electrode of the biofeedback monitoring device as a function of time.

Example 32 includes the subject matter of any of Examples 22-31, and further including providing a feedback to the user via at least one of electrode contact analysis device and the biofeedback monitoring device, wherein the feedback is based on the quality of the contact.

Example 33 includes the subject matter of any of Examples 22-32, and wherein the biofeedback monitoring device includes the electrode contact analysis device.

Example 34 includes an electrode contact analysis device comprising a processor; and a memory having stored therein a plurality of instructions that when executed by the processor cause the computing device to perform the method of any of Examples 22-33.

Example 35 includes one or more machine readable storage media comprising a plurality of instructions stored thereon that in response to being executed result in an electrode contact analysis device performing the method of any of Examples 22-33.

Example 36 includes an electrode contact analysis device to monitor contact between one or more electrodes of a biofeedback monitoring device and a user, the electrode contact analysis device comprising means for transmitting, by an electrode of an electrode contact analysis device, a body coupled communication (BCC) reference signal to a body of the user; means for receiving, from the biofeedback monitoring device, biofeedback signals received by an electrode of the biofeedback monitoring device; means for processing, by the electrode contact analysis device, the received biofeedback signals to produce a received BCC reference signal; and means for determining, by the electrode contact analysis device, a quality of a contact between the electrode of the biofeedback monitoring device and the user based on the received BCC reference signal.

Example 37 includes the subject matter of Example 36, and further including means for generating, by the electrode contact analysis device, the BCC reference signal including an identifier, wherein at least a portion of the identifier corresponds to at least one of the electrode contact analysis device and the electrode of the electrode contact analysis device.

Example 38 includes the subject matter of any of Examples 36 and 37, and wherein the means for determining the quality of the contact between the electrode and the user comprises means for comparing the received BCC reference signal and a threshold.

Example 39 includes the subject matter of any of Examples 36-38, and wherein the means for comparing the received BCC reference signal and a threshold comprises means for comparing a magnitude of the received BCC reference signal and a magnitude threshold.

Example 40 includes the subject matter of any of Examples 36-39, and further including means for generating a notification in response to the received BCC reference signal having a predetermined relationship with the threshold; and means for providing the notification to an operator of the electrode contact analysis.

Example 41 includes the subject matter of any of Examples 36-40, and wherein the means for generating the notification comprises means for activating a visual indicator of the electrode contact analysis device.

Example 42 includes the subject matter of any of Examples 36-41, and wherein the means for transmitting the BCC reference signal to the body of the user comprises transmitting the BCC reference signal to the body of the user via one of a capacitive coupled transmission and a galvanic coupled transmission.

Example 43 includes the subject matter of any of Examples 36-42, and further including means for detecting, by the electrode contact analysis device, a common mode change of the received BCC reference signal by the electrode of the biofeedback monitoring device; and means for subtracting, by the electrode contact analysis device, the common mode change from the received BCC reference signal to provide a compensated received BCC reference signal.

Example 44 includes the subject matter of any of Examples 36-43, and further including means for transmitting, by the electrode contact analysis device, a calibration command to compensate for a change of an environment condition detected by the electrode of the biofeedback monitoring device.

Example 45 includes the subject matter of any of Examples 36-44, and wherein the means for transmitting the calibration command comprises means for compensating for the change of the environment condition at the electrode of the biofeedback monitoring device as a function of time.

Example 46 includes the subject matter of any of Examples 36-45, and further including means for providing a feedback to the user via at least one of electrode contact analysis device and the biofeedback monitoring device, wherein the feedback is based on the quality of the contact.

Example 47 includes the subject matter of any of Examples 36-46, and wherein the biofeedback monitoring device includes the electrode contact analysis device.

## Claims

1. An electrode contact analysis device (120) for monitoring contact between one or more electrodes (116) of a biofeedback monitoring device (110) and a user (102), the electrode contact analysis device comprising:
a body area network, BAN, communication circuit (130) to transmit a body coupled communication, BCC, reference signal via a transmission electrode (138) of a plurality of transmission electrodes;
a communication circuit (132) to receive biofeedback signals from the biofeedback monitoring device, wherein the biofeedback signals comprise biofeedback signals received by the electrode of the biofeedback monitoring device;
a reference signal processing module (506) to process the received biofeedback signals to produce a received BCC reference signal from the received biofeedback signals;
an electrode contact determination module (508) to determine a quality of a contact between the electrode and the user based on the received BCC reference signal; and
a reference signal generation module (504) to generate the BCC reference signal, wherein the BCC reference signal comprises a non-biological signal that includes an identifier, and wherein the identifier identifies the transmission electrode of the plurality of transmission electrodes.

2. The electrode contact analysis device of claim 1, wherein the electrode contact determination module is to determine the quality of the contact between the electrode and the user based on a comparison of the received BCC reference signal and a threshold.

3. The electrode contact analysis device of claim 2, wherein the electrode contact determination module is to determine the quality of the contact between the electrode and the user based on a comparison of a magnitude of the received BCC reference signal and a magnitude threshold.

4. The electrode contact analysis device of claim 2, wherein the electrode contact determination module is further to generate a notification and provide the notification to an operator of the electrode contact analysis device in response to the received BCC reference signal having a predetermined relationship with the threshold.

5. The electrode contact analysis device of claim 1, wherein the electrode contact determination module is further to detect a common mode change of the received BCC reference signal and to subtract the common mode change from the received BCC reference signal to provide a compensated BCC reference signal, and wherein the electrode contact determination module is to determine the quality of the contact between the electrode of the biofeedback monitoring device and the user based on the compensated BCC reference signal.

6. The electrode contact analysis device of claim 1, wherein the BAN communication circuit is further to transmit a calibration command via the transmission electrode to compensate for a change of an environment condition detected by at least one of the one or more electrodes of the biofeedback monitoring device.

7. A method for monitoring contact between one or more electrodes of a biofeedback monitoring device and a user, the method comprising:
transmitting (702), by an electrode of a plurality of electrodes of an electrode contact analysis device, a body coupled communication, BCC, reference signal to a body of the user;
receiving (704), from the biofeedback monitoring device, biofeedback signals received by an electrode of the biofeedback monitoring device;
processing (712), by the electrode contact analysis device, the received biofeedback signals to produce a received BCC reference signal;
determining (718), by the electrode contact analysis device, a quality of a contact between the electrode of the biofeedback monitoring device and the user based on the received BCC reference signal; and
generating, by the electrode contact analysis device, the BCC reference signal including an identifier, wherein the identifier identifies the electrode of the plurality of electrodes of the electrode contact analysis device.

8. The method of claim 7, wherein determining the quality of the contact between the electrode and the user comprises comparing the received BCC reference signal and a threshold.

9. The method of claim 8, wherein comparing the received BCC reference signal and the threshold comprises comparing a magnitude of the received BCC reference signal and a magnitude threshold.

10. The method of claim 7, further comprising:
detecting, by the electrode contact analysis device, a common mode change of the received BCC reference signal by the electrode of the biofeedback monitoring device; and
subtracting, by the electrode contact analysis device, the common mode change from the received BCC reference signal to provide a compensated received BCC reference signal.

11. The method of claim 7, further comprising transmitting, by the electrode contact analysis device, a calibration command to compensate for a change of an environment condition detected by the electrode of the biofeedback monitoring device.

12. The method of claim 7, further comprising providing a feedback to the user via at least one of electrode contact analysis device and the biofeedback monitoring device, wherein the feedback is based on the quality of the contact.

13. One or more machine readable storage media comprising a plurality of instructions stored thereon that in response to being executed result in an electrode contact analysis device of any of claims 1-6 performing the method of any of claims 7-12.

## Patentansprüche

1. Elektrodenkontakt-Analysevorrichtung (120) zur Überwachung des Kontakts zwischen einer oder mehreren Elektroden (116) einer Biofeedback-Überwachungsvorrichtung (110) und einem Benutzer (102), wobei die Elektrodenkontakt-Analysevorrichtung umfasst:
eine Kommunikationsschaltung (130) für ein körpernahes Netz, BAN (Body Area Network), zum Übertragen eines Referenzsignals für die körpergekoppelte Kommunikation, BCC (Body Coupled Communication), über eine Übertragungselektrode (138) von mehreren Übertragungselektroden;
eine Kommunikationsschaltung (132) zum Empfangen von Biofeedback-Signalen von der Biofeedback-Überwachungsvorrichtung, wobei die Biofeedback-Signale Biofeedback-Signale umfassen, die von der Elektrode der Biofeedback-Überwachungsvorrichtung empfangen werden;
ein Referenzsignal-Verarbeitungsmodul (506) zum Verarbeiten der empfangenen Biofeedback-Signale, um aus den empfangenen Biofeedback-Signalen ein empfangenes BCC-Referenzsignal zu erzeugen;
ein Elektrodenkontakt-Bestimmungsmodul (508) zum Bestimmen der Qualität eines Kontakts zwischen der Elektrode und dem Benutzer basierend auf dem empfangenen BCC-Referenzsignal; und
ein Referenzsignal-Erzeugungsmodul (504) zum Erzeugen des BCC-Referenzsignals, wobei das BCC-Referenzsignal ein nichtbiologisches Signal umfasst, das eine Kennung enthält, und wobei die Kennung die Übertragungselektrode der mehreren Übertragungselektroden identifiziert.

2. Elektrodenkontakt-Analysevorrichtung nach Anspruch 1, wobei das Elektrodenkontakt-Bestimmungsmodul dazu dient, die Qualität des Kontakts zwischen der Elektrode und dem Benutzer basierend auf einem Vergleich des empfangenen BCC-Referenzsignals und eines Schwellenwerts zu bestimmen.

3. Elektrodenkontakt-Analysevorrichtung nach Anspruch 2, wobei das Elektrodenkontakt-Bestimmungsmodul dazu dient, die Qualität des Kontakts zwischen der Elektrode und dem Benutzer basierend auf einem Vergleich einer Größe des empfangenen BCC-Referenzsignals und eines Größenschwellenwerts zu bestimmen.

4. Elektrodenkontakt-Analysevorrichtung nach Anspruch 2, wobei das Elektrodenkontakt-Bestimmungsmodul ferner dazu dient, eine Benachrichtigung zu erzeugen und die Benachrichtigung in Reaktion darauf, dass das empfangene BCC-Referenzsignal ein vorbestimmtes Verhältnis zum Schwellenwert aufweist, an einen Bediener der Elektrodenkontakt-Analysevorrichtung bereitzustellen.

5. Elektrodenkontakt-Analysevorrichtung nach Anspruch 1, wobei das Elektrodenkontakt-Bestimmungsmodul ferner dazu dient, eine Gleichtaktänderung des empfangenen BCC-Referenzsignals zu erkennen und die Gleichtaktänderung von dem empfangenen BCC-Referenzsignal zu subtrahieren, um ein kompensiertes BCC-Referenzsignal bereitzustellen, und wobei das Elektrodenkontakt-Bestimmungsmodul dazu dient, die Qualität des Kontakts zwischen der Elektrode der Biofeedback-Überwachungsvorrichtung und dem Benutzer basierend auf dem kompensierten BCC-Referenzsignal zu bestimmen.

6. Elektrodenkontakt-Analysevorrichtung nach Anspruch 1, wobei die BAN-Kommunikationsschaltung ferner einen Kalibrierungsbefehl über die Übertragungselektrode überträgt, um eine Änderung eines Umgebungszustands, die von wenigstens einer der ein oder mehreren Elektroden der Biofeedback-Überwachungsvorrichtung erkannt wird, zu kompensieren.

7. Verfahren zur Überwachung des Kontakts zwischen einer oder mehreren Elektroden einer Biofeedback-Überwachungsvorrichtung und einem Benutzer, wobei das Verfahren umfasst:
Übertragen (702), durch eine Elektrode von mehreren Elektroden einer Elektrodenkontakt-Analysevorrichtung, eines BCC (körpergekoppelte Kommunikation)-Referenzsignals an einen Körper des Benutzers;
Empfangen (704), von der Biofeedback-Überwachungsvorrichtung, von Biofeedback-Signalen, die von einer Elektrode der Biofeedback-Überwachungsvorrichtung empfangen werden;
Verarbeiten (712), durch die Elektrodenkontakt-Analysevorrichtung, der empfangenen Biofeedback-Signale, um ein empfangenes BCC-Referenzsignal zu erzeugen;
Bestimmen (718), durch die Elektrodenkontakt-Analysevorrichtung, einer Kontaktqualität zwischen der Elektrode der Biofeedback-Überwachungsvorrichtung und dem Benutzer basierend auf dem empfangenen BCC-Referenzsignal; und
Erzeugen, durch die Elektrodenkontakt-Analysevorrichtung, des BCC-Referenzsignals, das eine Kennung enthält, wobei die Kennung die Elektrode der mehreren Elektroden der Elektrodenkontakt-Analysevorrichtung identifiziert.

8. Verfahren nach Anspruch 7, wobei das Bestimmen der Qualität des Kontakts zwischen der Elektrode und dem Benutzer umfasst, das empfangene BCC-Referenzsignal und einen Schwellenwert zu vergleichen.

9. Verfahren nach Anspruch 8, wobei das Vergleichen des empfangenen BCC-Referenzsignals und des Schwellenwerts umfasst, eine Größe des empfangenen BCC-Referenzsignals und einen Größenschwellenwert zu vergleichen.

10. Verfahren nach Anspruch 7, ferner umfassend:
Erkennen, durch die Elektrodenkontakt-Analysevorrichtung, einer Gleichtaktänderung des empfangenen BCC-Referenzsignals durch die Elektrode der Biofeedback-Überwachungsvorrichtung; und
Subtrahieren, durch die Elektrodenkontakt-Analysevorrichtung, der Gleichtaktänderung vom empfangenen BCC-Referenzsignal, um ein kompensiertes empfangenes BCC-Referenzsignal bereitzustellen.

11. Verfahren nach Anspruch 7, ferner umfassend das Übertragen, durch die Elektrodenkontakt-Analysevorrichtung, eines Kalibrierungsbefehls, um eine Änderung eines Umgebungszustands, die von der Elektrode der Biofeedback-Überwachungsvorrichtung erkannt wird, zu kompensieren.

12. Verfahren nach Anspruch 7, ferner umfassend das Bereitstellen einer Rückmeldung an den Benutzer über wenigstens entweder die Elektrodenkontakt-Analysevorrichtung und/oder die Biofeedback-Überwachungsvorrichtung, wobei die Rückmeldung auf der Qualität des Kontakts basiert.

13. Ein oder mehrere maschinenlesbare Speichermedien, die mehrere darauf gespeicherte Befehle umfassen, die in Reaktion auf die Ausführung bewirken, dass eine Elektrodenkontakt-Analysevorrichtung nach einem der Ansprüche 1-6 das Verfahren nach einem der Ansprüche 7-12 durchführt.

## Revendications

1. Dispositif d'analyse de contact d'électrode (120) pour surveiller le contact entre une ou plusieurs électrodes (116) d'un dispositif de surveillance par rétroaction biologique (110) et un utilisateur (102), le dispositif d'analyse de contact d'électrode comprenant :
un circuit de communication de réseau corporel, BAN, (130) pour transmettre un signal de référence de communication couplée au corps, BCC, par l'intermédiaire d'une électrode de transmission (138) d'une pluralité d'électrodes de transmission ;
un circuit de communication (132) pour recevoir des signaux de rétroaction biologique du dispositif de surveillance par rétroaction biologique, où les signaux de rétroaction biologique comprennent des signaux de rétroaction biologique reçus par l'électrode du dispositif de surveillance par rétroaction biologique ;
un module de traitement de signal de référence (506) pour traiter les signaux de rétroaction biologique reçus afin de produire un signal de référence BCC reçu à partir des signaux de rétroaction biologique reçus ;
un module de détermination de contact d'électrode (508) pour déterminer la qualité d'un contact entre l'électrode et l'utilisateur sur la base du signal de référence BCC reçu ; et
un module de génération de signal de référence (504) pour générer le signal de référence BCC, où le signal de référence BCC comprend un signal non biologique qui inclut un identificateur, et où l'identificateur identifie l'électrode de transmission de la pluralité d'électrodes de transmission.

2. Dispositif d'analyse de contact d'électrode selon la revendication 1, dans lequel le module de détermination de contact d'électrode doit déterminer la qualité du contact entre l'électrode et l'utilisateur sur la base d'une comparaison du signal de référence BCC reçu et d'un seuil.

3. Dispositif d'analyse de contact d'électrode selon la revendication 2, dans lequel le module de détermination de contact d'électrode doit déterminer la qualité du contact entre l'électrode et l'utilisateur sur la base d'une comparaison d'une amplitude du signal de référence BCC reçu et d'un seuil d'amplitude.

4. Dispositif d'analyse de contact d'électrode selon la revendication 2, dans lequel le module de détermination de contact d'électrode doit en outre générer une notification et fournir la notification à un opérateur du dispositif d'analyse de contact d'électrode en réponse au signal de référence BCC reçu ayant une relation prédéterminée avec le seuil.

5. Dispositif d'analyse de contact d'électrode selon la revendication 1, dans lequel le module de détermination de contact d'électrode doit en outre détecter un changement de mode commun du signal de référence BCC reçu et soustraire le changement de mode commun du signal de référence BCC reçu pour fournir un signal de référence BCC compensé, et où le module de détermination de contact d'électrode doit déterminer la qualité du contact entre l'électrode du dispositif de surveillance par rétroaction biologique et l'utilisateur sur la base du signal de référence BCC compensé.

6. Dispositif d'analyse de contact d'électrode selon la revendication 1, dans lequel le circuit de communication BAN doit en outre transmettre une commande d'étalonnage par l'intermédiaire de l'électrode de transmission pour compenser un changement de condition environnementale détecté par au moins une des une ou plusieurs électrodes du dispositif de surveillance par rétroaction biologique.

7. Procédé de surveillance du contact entre une ou plusieurs électrodes d'un dispositif de surveillance par rétroaction biologique et un utilisateur, le procédé comprenant les étapes suivantes :
transmettre (702), au corps de l'utilisateur, par une électrode d'une pluralité d'électrodes d'un dispositif d'analyse de contact d'électrode, un signal de référence de communication couplée au corps, BCC ;
recevoir (704), depuis le dispositif de surveillance par rétroaction biologique, des signaux de rétroaction biologique reçus par une électrode du dispositif de surveillance par rétroaction biologique ;
traiter (712), par le dispositif d'analyse de contact d'électrode, des signaux de rétroaction biologique reçus pour produire un signal de référence BCC reçu ;
déterminer (718), par le dispositif d'analyse de contact d'électrode, la qualité d'un contact entre l'électrode du dispositif de surveillance par biofeedback et l'utilisateur sur la base du signal de référence BCC reçu ; et
générer, par le dispositif d'analyse de contact d'électrode, le signal de référence BCC comprenant un identificateur, où l'identificateur identifie l'électrode de la pluralité d'électrodes du dispositif d'analyse de contact d'électrode.

8. Procédé selon la revendication 7, dans lequel la détermination de la qualité du contact entre l'électrode et l'utilisateur comprend de comparer le signal de référence BCC reçu et un seuil.

9. Procédé selon la revendication 8, dans lequel la comparaison du signal de référence BCC reçu et du seuil comprend de comparer une amplitude du signal de référence BCC reçu et un seuil d'amplitude.

10. Procédé selon la revendication 7, comprenant en outre les étapes suivantes :
détecter, par le dispositif d'analyse de contact d'électrode, un changement de mode commun du signal de référence BCC reçu par l'électrode du dispositif de surveillance par rétroaction biologique ; et
soustraire, par le dispositif d'analyse de contact d'électrode, le changement de mode commun du signal de référence BCC reçu pour fournir un signal de référence BCC reçu compensé.

11. Procédé selon la revendication 7, comprenant en outre de transmettre, par le dispositif d'analyse de contact d'électrode, une commande d'étalonnage pour compenser un changement de condition environnementale détecté par l'électrode du dispositif de surveillance par rétroaction biologique.

12. Procédé selon la revendication 7, comprenant en outre de fournir une rétroaction à l'utilisateur par l'intermédiaire d'au moins un dispositif parmi le dispositif d'analyse de contact d'électrode et le dispositif de surveillance par rétroaction biologique, où la rétroaction est basée sur la qualité du contact.

13. Un ou plusieurs supports de stockage lisibles par machine comprenant une pluralité d'instructions y étant stockées, qui, en réponse à leur exécution, conduisent à ce qu'un dispositif d'analyse de contact d'électrode de l'une quelconque des revendications 1 à 6 exécute le procédé de l'une quelconque des revendications 7 à 12.
